Europäisches Patentamt
European Patent Office
Office européen des brevets

(19)

(11) Publication number: **0 193 283 A1**

# (12) EUROPEAN PATENT APPLICATION

(21) Application number: 86300585.6

(22) Date of filing: 29.01.86

(51) Int. Cl.[4]: C 07 D 513/04
A 61 K 31/47
//(C07D513/04, 277:00, 221:00)

(30) Priority: 02.02.85 GB 8502690
14.05.85 GB 8512142

(43) Date of publication of application:
03.09.86 Bulletin 86/36

(84) Designated Contracting States:
BE CH DE FR GB IT LI NL

(71) Applicant: BEECHAM GROUP PLC
Beecham House Great West Road
Brentford Middlesex TW8 9BD(GB)

(72) Inventor: O'Hanlon, Peter John
11 Furzefield Road
Reigate Surrrey(GB)

(72) Inventor: Rogers, Norman Harold
22 Componts Lane
Horsham Sussex(GB)

(72) Inventor: Sime, Fiona Mary
30 Holmesdale Road North Holmwood
Dorking Surrey(GB)

(74) Representative: Lockwood, Barbara Ann
Beecham Pharmaceuticals Biosciences Research Centre
Great Burgh Yew Tree Bottom Road
Epsom Surrey KT18 5XQ(GB)

## (54) Thiazoloquinoline derivatives, process for their preparation and compositions containing them.

(57) Compounds of formula (I)

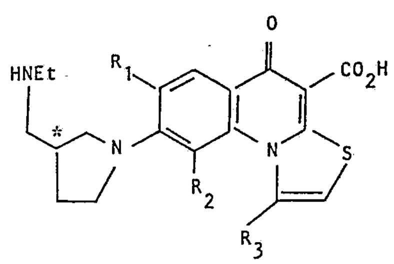

(I)

on a salt or ester thereof wherein $R_1$ is hydrogen or halogen, $R_2$ is hydrogen or halogen and $R_3$ is $C_{1-6}$ alkyl. These compounds have antibacterial and antimycoplasmal use.

EP 0 193 283 A1

TITLE MODIFIED

## NOVEL COMPOUNDS

This invention relates to novel substituted carboxylic acid derivatives, their preparation and use, and in particular to novel quinolone derivatives. These compounds have antibacterial and/or antimycoplasmal activity and are therefore of use in the treatment of bacterial infection in humans and animals caused by a wide range of organisms. In particular these compounds are of use in the treatment of mastitis in cattle, and for mycoplasma injections in animals such as turkeys, chickens, pigs and cattle.

According to the present invention there is provided a compound of formula (I)

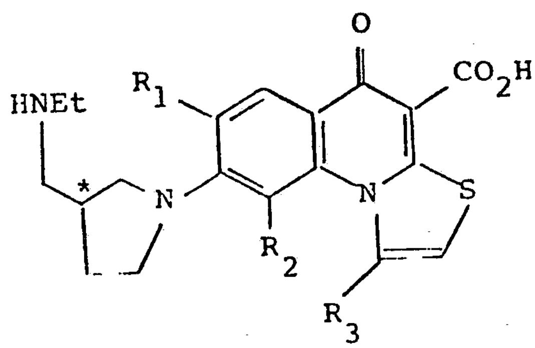

(I)

or a salt or ester thereof; wherein $R_1$ is hydrogen or halogen, $R_2$ is hydrogen or halogen; and $R_3$ is $C_{1-6}$ alkyl.

The term 'halogen' refers to fluorine, chlorine, bromine and iodine. Suitably $R_1$ is fluorine. Suitably $R_2$ is hydrogen or fluorine. Preferably $R_2$ is hydrogen.

The alkyl group may be straight or branched chain alkyl groups containing from 1 to 6 carbon atoms. Preferably $R_3$ is methyl.

The carbon atom marked * in formula (I) is an asymmetric carbon and exists in two isomeric forms. The present invention includes both isomers of formula (I) together with mixtures thereof.

Since the compounds of formula (I) are primarily intended for use as pharmaceuticals, salts and esters thereof are preferably pharmaceutically acceptable.

Suitable pharmaceutically acceptable salts of the compounds of formula (I) include acid addition salts, metal salts, in particular alkali metal salts such as sodium or potassium, or salts with strong organic bases for example those with lower alkylamines such as triethylamine, or with guanidine or tetramethylguanidine, or quaternary ammonium salts such as t-butyl ammonium salts.

Suitable acid addition salts of compounds of formula (I) include pharmaceutically acceptable inorganic salts such as the sulphate, nitrate, phosphate, hydrochloride and hydrobromide and pharmaceutically acceptable organic acid addition salts such as tartrate, maleate, citrate, methane-sulphonate, p-toluene-sulphonate, α-glycerophosphate, and glucose-1-phosphate.

Examples of suitable pharmaceutically acceptable ester groups include those which are *in-vivo* hydrolysable in that they break down readily in the human or animal body to leave the parent acid or its salt.
The compounds of formula (I) can be prepared by reacting a compound of formula (II)

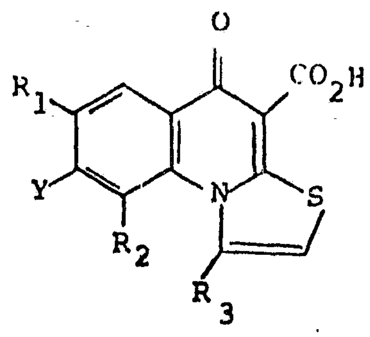

(II)

wherein $R_1$, $R_2$ and $R_3$ are as defined in relation to formula (I) and Y is a leaving group; with N-ethyl-3-pyrrolidinemethanamine.

Suitably Y is halogen such as fluorine or alkane sulphonate such as mesylate, tosylate or trifluoromethanesulphonate.

Preferably Y is fluorine.

The reaction is suitably carried out in an inert organic solvent such as acetonitrile at an elevated temperature for example of from 20-200°C.

Compounds of formula (II) can be prepared as described in European Published Patent Application No. 0058392.

N-ethyl-3-pyrrolidinemethanamine is a known compound.

Some of the human and veterinary diseases either caused by mycoplasma species or in which they play a prominent role, and against which compounds of this invention are effective, are as follows:

Avian

| | | |
|---|---|---|
| M. gallisepticum | - | chronic respiratory diseases (air sacculitis) of chickens and turkey |

Bovine

| | | |
|---|---|---|
| M. bovis | - | mastitis, respiratory disease and arthritis of cattle |
| M. dispar | - | calf pneumonia |

Porcine

| | | |
|---|---|---|
| M. hyopneumoniae | - | enzootic pneumonia of pigs |
| M. hyorhinis<br>M.hyosyoviae | - | arthritis in pigs |

| | | |
|---|---|---|
| Human | - | primary atypical pneumonia |

Compounds of the present invention are particularly useful in the treatment of enzootic pneumonia in animals such as pigs, cattle and sheep.

The present invention also provides a pharmaceutical composition which comprises a compound of this invention and a pharmaceutically acceptable carrier.

The compositions of the invention include those in a form adapted for oral, topical or parenteral use and may be used for the treatment of the infection in mammals including humans.

The composition may be formulated for administration by any route, such as oral, topical or parenteral. The compositions may be in the form of tablets, capsules, powders, granules, lozenges, creams or liquid preparations, such as oral or sterile parenteral solutions or suspensions.

For veterinary use the composition may also be a dispersion or a solution of the drug in a suitable vehicle for use with an oral doser (this is a well known item of farm equipment, basically comprising a liquid reservoir, a mouthpiece adapted for insertion into animals mouths, and a pump mechanism whereby unit doses can be ejected from the reservoir through the mouthpiece). Conveniently the drug may be administered from an oral doser as an aqueous solution. Alternatively, the vehicle will be an oil or water based cream to ensure homogeneity of the unit doses administered.

The invention, therefore, also provides an oral doser containing a multi-dose of the drug in a veterinarily acceptable vehicle.

The drugs of the invention may also be added to the animal feed or drinking water. Thus the invention also provides animal feed or animal drinking water containing a compound of formula (I). It will be convenient to formulate these animal feed and drinking water compositions with a multi-dose of the drug so that the animal takes in an appropriate quantity of the drug along with its diet. It will also be convenient to present the composition of the invention as a pre-mix for addition to the feed or drinking water.

With human babies or young animals, a particularly useful technique is to blend their milk with the drugs of this invention.

In this instance 'topical administration' also includes local administration to the internal surfaces of mammary glands of cattle, for example during the treatment of mastitis by intra-mammary administration.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams. The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

For intramammary administration, the active ingredients may be incorporated into any suitable veterinary carrier. For example, the ingredients may be suspended in an oily vehicle, with an appropriate thickening agent.

With human babies or young animals, a particularly useful technique is to blend their milk with the drugs of this invention.

In this instance 'topical administration' also includes local administration to the internal surfaces of mammary glands of cattle, for example during the treatment of mastitis by intra-mammary administration.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams. The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

For intramammary administration, the active ingredients may be incorporated into any suitable veterinary carrier. For example, the ingredients may be suspended in an oily vehicle, with an appropriate thickening agent.

The oily vehicle may be a mineral oil, or a vegetable oil such as arachis oil, sesame oil, peanut oil, corn oil, cottonseed oil, soyabean oil, olive oil, or fractionated coconut oil.

The physical properties of the composition and the release rate of active ingredient may be varied by making an appropriate choice of oily vehicle and optional additives therefore. For example when a fast milk out is required for therapy of lactating cows, then an emulsifying agent may be included in the composition to hasten the mixing of the composition with the aqueous secretions in the udder; alternatively the base described in West Germany Offenlegungsschrift No. 26 35 476 may be used, namely fractionated coconut oil. If on the other hand slow release is required for

the therapy or prophylaxis of dry cows, then suitably a more hydrophobic oil vehicle which has been more strongly gelled with a gelling agent, such as aluminium stearate, is used. Thickening agents such as Thixcin R and silica may also be included in the compositions if so desired, and when present will normally represent 0.1 to 8%, more suitably 1 to 5% of the composition by weight.

For such administration, the chosen compositions will be filled into the tubes or syringe packs of the conventional type for intramammary administration, i.e. provided with a cannula nozzle for insertion into the teat to allow extrusion directly into the mammary gland via the streak canal.

Tablets and capsules for administration may be in unit dose presentation form, and may contain conventional excipients such as binding agents, for example syrup, acacia, gelatin, sorbitol, tragacanth, or polyvinylpyrollidone: fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl

cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parental suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The dosage of the compound will depend upon the nature of the infection being treated, as well as the severity of the condition. Generally the dosage for humans or mammals will be within the range of 0.1 to 100 mg/kg per day, preferably from 0.5 to 25 mg/kg per day.

Ruminant animals such as cows may be treated with an amount of a compound of formula I effective to treat or prevent mastitis. The precise dosage will depend upon various factors including whether the animal is lactating or dry and the type of carrier (if any) employed. For most cases, an effective treatment comprises administration of from 1 to 3 doses of from 50-1000mg of a compound of formula (I) at intervals of from 12 to 48 hours to each teat of the animal.

The present invention also includes a method of treating bacterial infections in humans and animals which comprises the administration of a therapeutically effective amount of compound of formula (I).
The following example illustrates the invention.

Example 1

8-[3-[(Ethylamino)methyl]-1-pyrrolidinyl]-7-fluoro -1-methyl-5-oxo-5H-thiazolo[3,2-a]-quinoline-4-carboxylic acid

A mixture of 7,8-difluoro-1-methyl-5-oxo-5H-thiazole (3,2-a)-quinoline-4-carboxylic acid (295mg 1mmol), N-ethyl-3-pyrrolidinemethanamine (138mg, 1.1mmol) and 1,8-diazabicyclo[5.4.0] undec-7-ene (0.15ml, 1mmol) were refluxed in acetronitrile (10ml) for 1.5h. The reaction mixture was then cooled, the solid filtered off, washed with ether, and dried to yield the title compound as a white solid (251mg, 62%) m.p. 254-256$^{o}$C; γmax (KBr) 1680, 1630, 1600 $cm^{-1}$; λmax(EtOH) 304nm (ε9,508) and 365 (12,912); δH($CF_3CO_2D$) 1.51 (3H,t, $CH_2C\underline{H}_3$), 3.25 (3H,s,1-$CH_3$), 1.9-4.3(11H,m, 7 x pyrrolidinyl-H, $C\underline{H}_2NC\underline{H}_2$) 7.60 (1H,s,2-H), 7.83 (1H,d,9-H), 8.24(1H,d,6-H).

The antibacterial activity of the compound is demonstrated by the following representative MIC data:

| | MIC(μg/ml) - agar |
|---|---|
| S.aureus M12 | 0.12 |
| S.agalactiae SPAI | 1 |
| S.dysgalactiae FRO 7A | 0.5 |

The antimyccoplasmal activity of the compound is demonstrated in Table 1.

Table 1

In vitro myccoplasmal activity of the compound of example 1.

| | | MIC ug/l |
|---|---|---|
| M. hyopneumoniae x 5 strains | | 0.001 |
| M. hyorhinis | ATCC 23234 | 0.05 |
| M. hyosynoviae | ATCC 25591 | 0.05 |
| M. bovis | NCTC 10131 | 0.1 |
| M. dispar | NCTC 10125 | 0.025 |
| M. gallisepticum | S6 | 0.025 |
| M. synoviae | ATCC 25204 | 0.1 |
| M. iowae | B11 | 0.025 |
| M. meleagridis | 17529 | 0.025 |
| M. pneumoniae x vs 9 strains | | 0.13 |

MIC's where determined either on Friis' agarose medium supplemented into L-cysteine and NAD, or on $SP_4$ medium solidified with 0.65% agarose.

0193283

CLAIMS

1. A compound of formula (I)

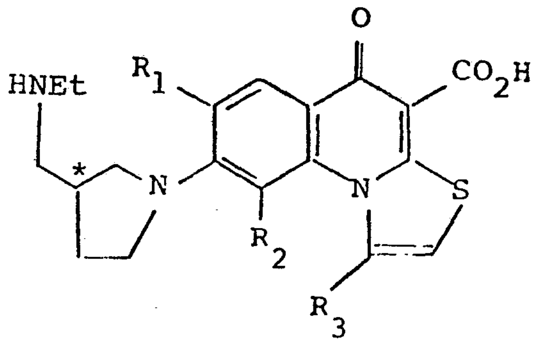

(I)

or a salt or ester thereof;
in which $R_1$ is hydrogen or halogen,
$R_2$ is hydrogen or halogen;
and $R_3$ is $C_{1-6}$ alkyl.

2. A compound as claimed in claim 1 in which $R_1$ is fluorine.

3. A compound as claimed in claim 1 or claim 2 in which $R_2$ is hydrogen or fluorine.

4. A compound as claimed in any one of claims 1 to 3 in which $R_3$ is methyl.

5. A process for producing a compound of formula (I) as claimed in claim 1 which process comprises reacting a compound of formula (II)

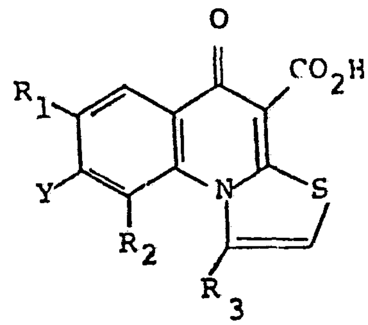

(II)

wherein $R_1$, $R_2$ and $R_3$ are as defined in relation to formula (I) and Y is a leaving group; with N-ethyl-3-pyrrolidinemethanamine.

6. A process as claimed in claim 5 in which Y is a halogen or alkane sulphonate.

7. A process as claimed in claim 5 or claim 6 in which Y is fluorine.

8. A pharmaceutical or veterinary composition comprising a compound of formula (I) as defined in claim 1 and a pharmaceutically or veterinarily acceptable carrier.

0193283

European Patent Office

# EUROPEAN SEARCH REPORT

Application number

EP 86 30 0585

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | EP-A-0 058 392 (NIPPON) * Claim 1; pages 50-59 * | 1,8 | C 07 D 513/04<br>A 61 K 31/47 //<br>(C 07 D 513/04<br>C 07 D 277:00<br>C 07 D 221:00 ) |
| Y | EP-A-0 106 489 (WARNER-LAMBERT) * Claim 1; page 26, lines 18-19; pages 27-36 * | 1,8 | |
| | | | TECHNICAL FIELDS SEARCHED (Int. Cl.4) |
| | | | C 07 D 513/00<br>A 61 K 31/00 |

The present search report has been drawn up for all claims

| Place of search | Date of completion of the search | Examiner |
|---|---|---|
| THE HAGUE | 13-05-1986 | ALFARO I. |

CATEGORY OF CITED DOCUMENTS

X particularly relevant if taken alone
Y particularly relevant if combined with another document of the same category
A technological background
O non-written disclosure
P intermediate document

T : theory or principle underlying the invention
E : earlier patent document, but published on, or after the filing date
D document cited in the application
L document cited for other reasons
& member of the same patent family, corresponding document

EPO Form 1503 03 82